# EUROPEAN PATENT SPECIFICATION

(11) **EP 4 544 452 B1**
(45) Date of publication and mention of the grant of the patent: **18.11.2026**
(21) Application number: 23738164.5
(22) Date of filing: 22.06.2023
(51) Int. Cl.: G06N 3/045, G06N 3/04

(54) **PHYSIOLOGICAL CHARACTERISTIC WAVEFORM CLASSIFICATION WITH EFFICIENT DEEP NETWORK SEARCH**
KLASSIFIZIERUNG PHYSIOLOGISCHER CHARAKTERISTISCHER WELLENFORMEN MIT EFFIZIENTER SUCHE NACH TIEFEN NETZWERKEN
CLASSIFICATION DE FORME D'ONDE DE CARACTÉRISTIQUE PHYSIOLOGIQUE AVEC RECHERCHE DE RÉSEAU PROFOND EFFICACE

(30) Priority: 22.06.2022 US 202263354502 P
(43) Date of publication of application: 30.04.2025
(73) Proprietor: Drägerwerk AG & Co. KGaA, 23558 Lübeck (DE)
(72) Inventor: PIMENTA, Anne-severine Lima, Windham, New Hampshire 03087 (US); WANG, Xianju, Bedford, Massachusetts 01730 (US)
(74) Representative: Haseltine Lake Kempner LLP
(86) International application number: PCT/IB2023/056437
(87) International publication number: WO 2023/248166

(56) References cited:
- WO-A1-2021/143883
- US-A1- 2022 351 019
- ZHANG YONGAN YZ87@RICE EDU ET AL: "RT-RCG: Neural Network and Accelerator Search Towards Effective and Real-time ECG Reconstruction from Intracardiac Electrograms", ACM JOURNAL ON EMERGING TECHNOLOGIES IN COMPUTING SYSTEMS (JETC), ACM, 2 PENN PLAZA, SUITE 701 NEW YORK NY 10121-0701 USA, vol. 18, no. 2, 16 March 2022 (2022-03-16), pages 1 - 25, XP058678149, ISSN: 1550-4832, DOI: 10.1145/3465372

## Description

### CROSS-REFERENCE TO RELATED APPLICATIONS

The priority and earlier effective filing date of U.S. Application Serial No. 63/354,502, filed June 22, 2022, is hereby claimed for all purposes, including the right of priority.

### TECHNICAL FIELD

The present disclosure pertains to artificial neural networks and, more particularly, to a neural architecture search as may be used in designing an artificial neural network.

### DESCRIPTION OF THE RELATED ART

This section of this document introduces information about and/or from the art that may provide context for or be related to the subject matter described herein and/or claimed below. It provides background information to facilitate a better understanding of the various aspects of the that which is claimed below. This is a discussion of "related" art. That such art is related in no way implies that it is also "prior" art. The related art may or may not be prior art. The discussion in this section of this document is to be read in this light, and not as admissions of prior art.

The physiological state of a patient in a clinical setting is frequently represented by monitoring one or more physiological characteristics of the patient over time. The monitoring usually includes capturing data using sensors, the captured data representing each physiological characteristic. Because the data is representative of a physiological characteristic, the data may be referred to as "physiological data".

In one common example known as electrocardiography, a number of electrodes are placed at predetermined points on the patient's body. Each sensor produces a voltage signal that is captured and graphed. The graph, or electrocardiogram ("ECG", or "EKG"), illustrates in graphical form the magnitude of the voltage signal over time. A trained clinician or technician can then use this graphical representation to determine how "normally" or "non-normally" the patient's heart is beating. Thus, in this context, the captured physiological data is representative of the patient's heartbeat.

The data captured by such monitoring is typically ordered by at least the time of acquisition and an attribute of the physiological characteristic as represented in the data. In the ECG example above, the data is ordered not only by time of acquisition and the magnitude of the voltage, but also by the sensor through which the data was acquired. The voltage acquired by an individual sensor can be rendered for human perception as a graph, or plot, of the voltage magnitude over time referred to as a "waveform". It is also common to collectively refer to the physiological data as a "waveform". The result of the ECG, then, is a set of waveforms reflecting the patient's heartbeat.

It was mentioned above relative to the ECG that a trained clinician or technician may analyze "waveforms" to determine certain aspects of the patient's condition captured in the physiological data. For a variety of reasons, the art has turned to "intelligent machines", or computing machines employing various kinds of artificial intelligences, to perform this analysis or evaluation. However, like the clinician or technician, the intelligent machine must be trained to perform this analysis.

One kind of artificial intelligence is a "neural network", sometimes also called an "artificial neural network", or "ANN". One technique sometimes used to train an intelligent machine, and more specifically an artificial neural network, is a "neural architecture search", or "NAS". An NAS is one technique for automating the design of artificial neural networks. NAS has been used to design ANNs that are on par or outperform hand-designed ANNs.

WO 2021/143883 discloses an adaptive search method and apparatus for a neural network.

Artificial neural networks can be large and complex, thereby consuming a lot of time and effort to design. Even an automated NAS may take a lot of time and computing resources. For example, an NAS may consume anywhere between 100 to a few thousand processor-hours. Outcome accuracy is also prized, and greater outcome accuracy is generally preferred over lesser, all else being equal. Note, however, that the design process may involve tradeoffs. The art is therefore receptive to new approaches to NAS that improve outcome accuracy, computational resource consumption, and design time.

### BRIEF DESCRIPTION OF THE DRAWINGS

Illustrative embodiments of the subject matter claimed below will now be disclosed. In the interest of clarity, not all features of an actual implementation are described in this specification. It will be appreciated that in the development of any such actual implementation, numerous implementation-specific decisions must be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.
Figure 1 conceptually depicts an artificial neural network designed in accordance with an embodiment of the present disclosure.
Figure 2A-Figure 2B illustrate one particular embodiment of a method for use in designing the artificial neural network of Figure 1.
Figure 3 schematically depicts one particular embodiment of a computing apparatus by which the method of Figure 2A-Figure 2B may be performed in the design of the artificial neural network of Figure 1.
Figure 4A-Figure 4B illustrate one particular embodiment of a method for designing an artificial neural network in accordance with the present disclosure.
Figure 5 conceptually depicts an artificial neural network designed by the method of Figure 4A-Figure 4B.
Figure 6, Figure 7A-Figure 7E, and Figure 8A-Figure 8B illustrate assorted operators as may be used in the implementation of the artificial neural network of Figure 1 or the artificial neural network of Figure 5.
Figure 9 schematically depicts one particular embodiment of a computing apparatus by which the method of Figure 4A-Figure 4B may be performed in the design of the artificial neural network of Figure 5.
Figure 10 presents a workflow performed by the computing apparatus of Figure 9 to implement the method of Figure 4A-Figure 4B to design the artificial neural network of Figure 5.
Figure 11 illustrates an ECG acquisition of input sample beats.
Figure 12 represents an acquired ECG waveform.
Figure 13A-Figure 13B present an example N beat.
Figure 14A-Figure 14B present an example S beat.
Figure 15A-Figure 15B present an example B beat.
Figure 16A-Figure 16B present an example V beat.
Figure 17A-Figure 17B present an example F beat.
Figure 18A-Figure 18B present an example Q beat.

### DETAILED DESCRIPTION

The present disclosure provides an NAS technique that yields superior outcome accuracy and also may reduce both computational resource consumption, and design time relative to many conventional techniques. The disclosed NAS technique therefore improves the operational performance of the computing resources with which the underlying ANN is designed and operated. The disclosed NAS technique is furthermore embedded in a practical application-namely, the design of an ANN. Still further, the NAS can be used to design an ANN that is optimized to analyze a certain kind of output. One such embodiment is discussed further below.

In one embodiment, a method for use in designing an artificial neural network, comprises: performing a two-step neural architecture search, and selecting for each respective layer the operators that yield the maximum weighted output for each respective layer in the two-step neural architecture search. The two-step neural architecture search may include training the plurality of operators within each of the layers; and training a plurality of weights, each weight being applied to a respective combination of operators within a respective layer.

In another embodiment, a method for designing an artificial neural network, comprises: creating a deep neural network comprising a plurality of layers, each of the layers comprising a plurality of operators; accessing a plurality of sample inputs; performing a two-step neural architecture search using the accessed sample inputs; and selecting for each respective layer the operators that yield the maximum weighted output for each respective layer in the two-step neural architecture search. The two-step neural architecture search may include: training the plurality of operators within each of the layers; and training a plurality of weights, each weight being applied to a respective combination of operators within a respective layer.

In yet another embodiment, a method for creating a deep neural network includes defining a deep neural network; obtaining a plurality of sample inputs; and training the deep neural network on the sample inputs. Defining the deep neural network may include defining a number of layers, each layer receiving a layer input and generating a layer output, each layer comprising a plurality of operators, each operator including a plurality of operator parameters; and for each of the plurality of operators within each layer, pairing each operator with an instance of itself and an instance of each other operator within the layer, each instance of each operator receiving a layer input from the previous layer and generating an operator output; summing the operator outputs of each pair of operators; and weighting the summed operator outputs, the sum of the weights equaling 1, to generate a plurality of layer outputs. Training the deep neural network may include a first pass in which each of the weights is fixed in order to train the operator parameters; a second pass in which the operator parameters are fixed in order to train the weights; selecting for each layer the paired operators that yield the maximum weighted output to provide the layer output; and deselecting for each layer each set of paired operators that does not yield the maximum weighted output.

In still other embodiments, a computing apparatus, comprises: a processor-based resource; and a memory electronically communicating with the processor-based resource. The memory may be encoded with instructions that, when executed by the processor-based resource, perform the methods set forth herein.

Still other embodiments include a non-transitory, computer readable medium encoded with instructions that, when executed by a processor-based resource, perform the methods set forth herein.

One or more specific embodiments of the presently claimed subject matter will be described below in association with the drawings appended hereto. The present presently claimed subject matter is not limited to the embodiments and illustrations contained herein, but includes modified forms of those embodiments including portions of the embodiments and combinations of elements of different embodiments as come within the scope of the appended claims. In the development of any such actual implementation, as in any engineering or design project, numerous implementation-specific decisions must be made to achieve the developers' specific goals, such as compliance with system-related and business related constraints, which may vary from one implementation to another. Moreover, such a development effort might be complex and time consuming, but would nevertheless be a routine undertaking of design, fabrication, and manufacture for those of ordinary skill having the benefit of this disclosure.

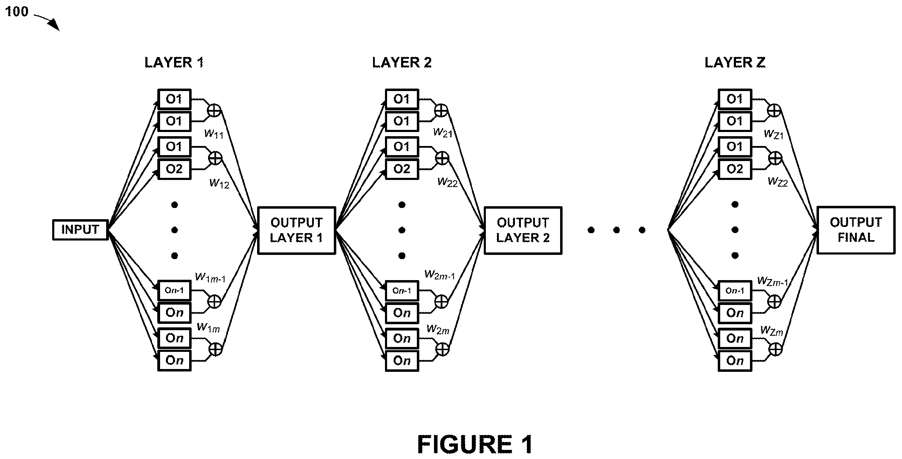

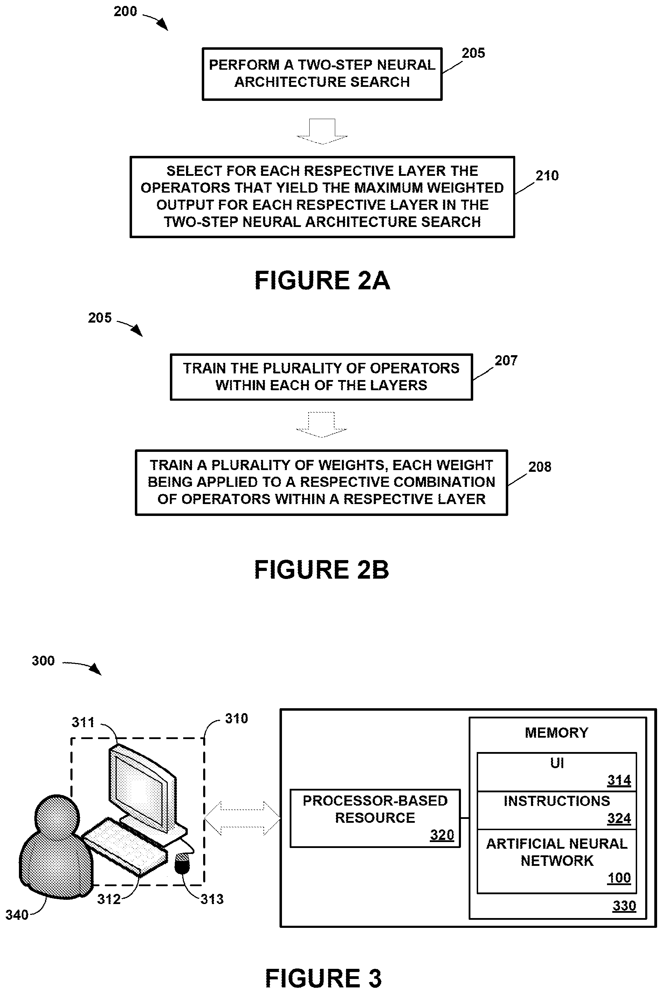

Turning now to the drawings, Figure 1 conceptually depicts an artificial neural network 100 designed in accordance with an embodiment of the present disclosure and Figure 2A-Figure 2B illustrate one particular embodiment of a method 200 for use in designing the artificial neural network of Figure 1. This particular embodiment assumes that the artificial neural network 100 has already been designed in its broad form-i.e., as shown in Figure 1. Thus, the number of layers, the number of operators per layer, the identity of the layers, *etc.* has already been determined.

More particularly, the artificial neural network 100 comprises *Z* layers, designated layer 1-layer *Z.* Each layer comprises *n* operators, designated O1-O*n*. Within each layer, each operator is paired with itself and each of the other operators. Thus, operator O1 is paired with itself and each other operator O2-O*n* in an act generating *m* pairs of operators. (Note that *m = n*!*+n*.) Each of the *m* pairs in each layer is weighted individually. In the nomenclature of Figure 1, the weight *w*₁₁ is the first weight in the first layer, the weight *w₁₂* is the second weight in the first layer, and so on. The sum of the *m* weights in each layer is, in this particular embodiment, equal to 1.

The flow of information in the artificial neural network 100 is from left to right in Figure 1 as indicated by the arrows therein. Each of the *Z* layers has an input and an output. The input for each layer is the output of the immediately preceding layer except for layer 1. The input to layer 1 comprises a plurality of samples, the nature and identity of which will depend on the end use of artificial neural network 100. The output of layer *Z* is a classification of any given input sample having just been processed through the artificial neural network 100.

The neural architecture search disclosed herein determines which of the *m* pairs of operators will be selected in each of the *Z* layers to be used in the design of the artificial neural network 100. One embodiment of this technique is the method 200 illustrated in Figure 2A-Figure 2B. The method 200 is for use in designing an artificial neural network, such as the artificial neural network 100 in Figure 1. The method 200 begins (at 205) by performing a two-step neural architecture search, illustrated in Figure 2B. The two-step neural architecture search (at 205) may include, as shown in Figure 2B, training (at 207) the plurality of operators within each of the layers and training (at 208) a plurality of weights, each weight being applied to a respective combination of operators within a respective layer. Returning to Figure 2A, the method 200 continues (at 210) by selecting for each respective layer the operators that yield the maximum weighted output for each respective layer in the two-step neural architecture search.

As noted above, a neural architecture search is an automated process. Figure 3 schematically depicts one particular embodiment of a computing apparatus 300 by which the method of Figure 2A-Figure 2B may be performed in the design of the artificial neural network of Figure 1. The computing apparatus 300 may be, for instance, a workstation. The computing apparatus 300 generally comprises, in this particular embodiment, a user interface 310, a processor-based resource 320, and a memory 330. The user interface 320 includes a display 311, one or more peripheral devices such as a keyboard 312 and/or mouse 313, and a software component ("UI") 314 residing on the memory 330. The UI software component 314 is executed by the processor-based resource 320 to provide a presentation (not separately shown) on the display 311 with which a user 340 may interact using the peripheral component(s).

The processor-based resource 320 executes machine executable instructions 324 residing in the memory 330 to perform the functionality of the technique described herein. The instructions 324 may be embedded as firmware in the memory 330 or encoded as routines, subroutines, applications, *etc.* The memory 330 is a computer readable non-transitory, computer readable medium and may be local or remote.

Accordingly, in the illustrated embodiment, the computing apparatus 300 performs the software-implemented functionality of the presently disclosed technique. More particularly, the processor-based resource 320 executes the instructions 324, both shown in Figure 3, to perform the programmed functionality of the disclosed neural architecture search technique disclosed herein. The neural architecture search technique may be invoked by the user 340 through the user interface 310 as a part of the design process for the artificial neural network 100.

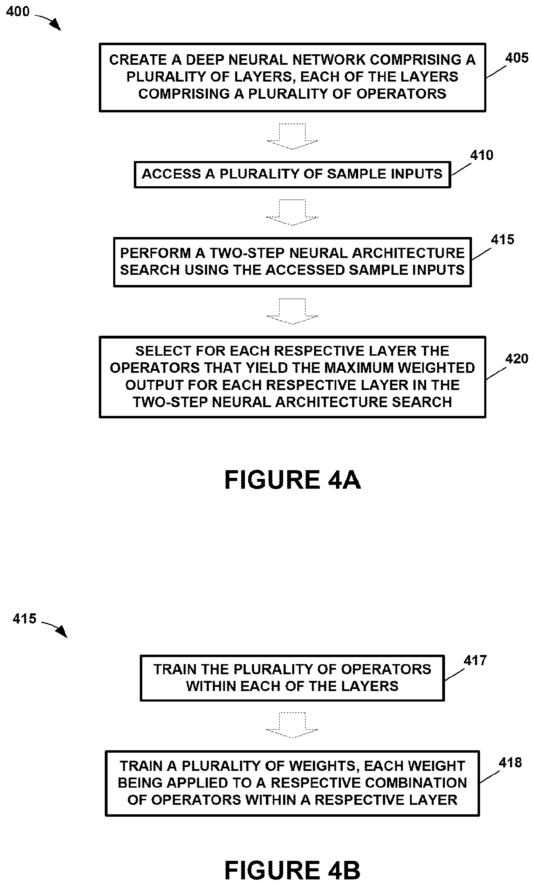

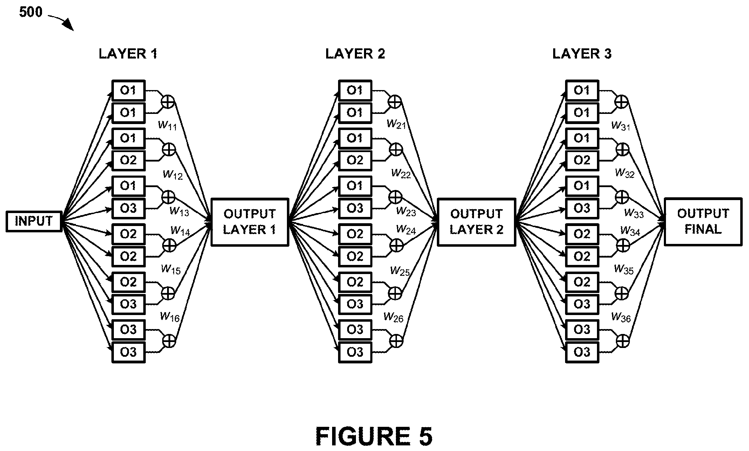

The neural architecture search described above may be set in a broader context of designing the artificial neural network as a whole. To that end, Figure 4A-Figure 4B illustrate a method 400 for designing an artificial neural network. For purposes of illustration, the method 400 will be discussed in the context of the artificial neural network 500 shown in Figure 5. The artificial neural network 500 is a kind known as a deep neural network and will be so-referenced moving forward.

The method 400 begins by creating (at 405) a deep neural network, for example, the deep neural network 500. The deep neural network 500 comprises a plurality of layers, each of the layers comprising a plurality of operators. More specifically, the deep neural network 500 comprises three layers, each of layer 1-layer 3 further comprising three operators, 01-03. Note that in each of the layers each operator is paired with itself as well as each of the other two operators. Thus, each of layer 1-layer 3 comprises 12 pairings of operators. Each pairing is summed, weighted, and transmitted as a part of the output for the respective layer.

Stated more technically, each layer is a two-branch structure mapping from one input tensor (*e.g.,* the output of the previous layer) to one output tensor. This can be described as input, O1, O2, addition. Addition is the operator to combine the individual outputs to form a block's output tensor. The pairings of operators shown in Figure 5 are all candidates to be the block that becomes the layer in the finished design of the artificial neural network. The two-step neural architecture search disclosed herein determines which of the candidates is selected for the finished design.

Those in the art having the benefit of this disclosure will appreciate that the design of the artificial neural network in any given embodiment may be implementation specific. Implementations of the artificial neural network may vary in both the number of layers and the number of operators. In one embodiment, for example, the artificial neural network comprises eight layers, each layer including 13 operators in 13!+13 pairings. Using the nomenclature established above, *Z*=8, *n*=13, and *m*=13!+13 in this particular implementation.

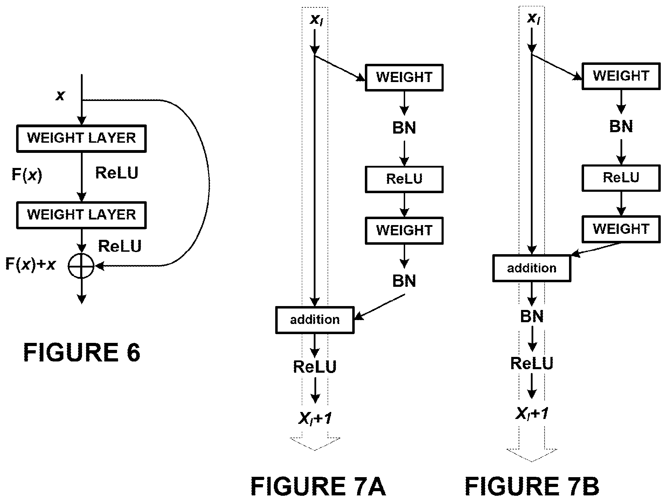

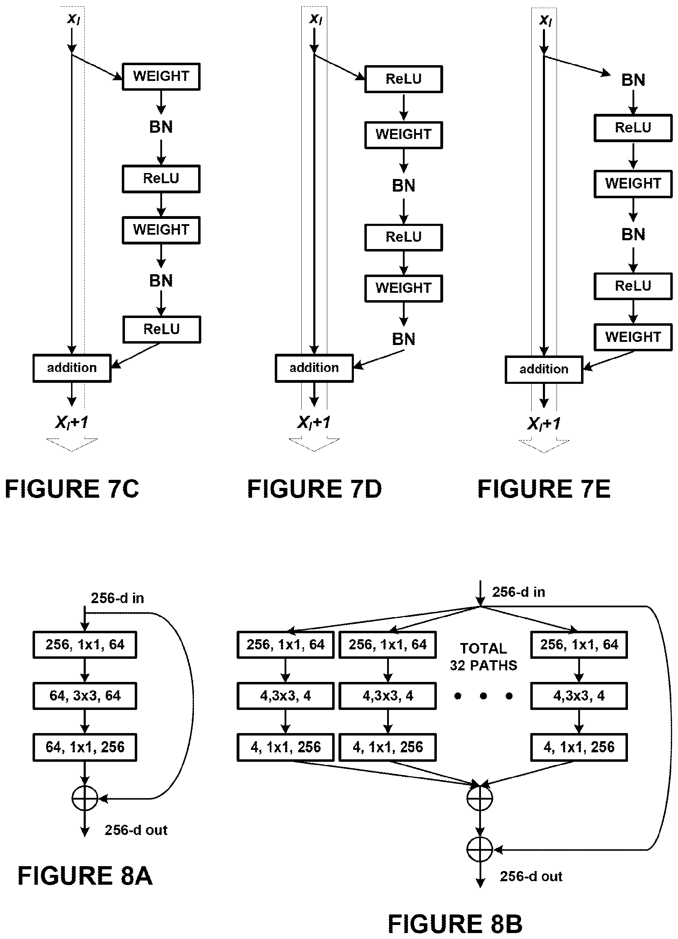

The operators O1-ON may be selected from any of a variety of operators. Figure 6, Figure 7A-Figure 7E, and Figure 8A-Figure 8B illustrate assorted operators as may be used in the implementation of the artificial neural network of Figure 1 and/or Figure 5. The parameter of the filter is the weight, weight layer, or coefficient of the filter. Figure 6 illustrates application of a rectified linear unit ("ReLU"). Figure 7A-Figure 7E illustrate operations including one or more rectified linear units ("ReLUs") and batch normalizations ("BN") in varying combinations and orderings. Figure 8A-Figure 8B illustrate two implementations of a variation known as ResNet of an operator known in the art as residual network ("ResNet"). ResNet is one type of convolutional neural network, which is one type of deep neural network. The implementations shown in Figure 8A-Figure 8B can use different parameters (e.g. number of filters). For example, instead of 256, the number of filters may be 32, 64, or 128. Still other operators known to the art (*e.g.,* "Maxpooling") may also be used.

Those in the art having the benefit of this disclosure will appreciate that the choice of operators, their ordering, *etc.* will depend upon the purpose for which the artificial neural network is to be used. In some embodiments, the artificial neural networks 100, 500, are used in a clinical setting to classify waveforms representative of a patient's physiological characteristic. This usage will be discussed further below.

Returning to Figure 4A, after creating (at 405) the deep neural network, the method 400 then continues by accessing (at 410) a plurality of sample inputs. In the illustrated embodiment, the sample inputs are "labeled inputs" and, thus, the training discussed below is a form of what is known as "supervised learning". For example, as will be discussed further below, the sample inputs are waveforms representative of a patient's individual heartbeat. These are sometimes called "beats" in the respective art. These beats are classified as set forth in Table 1 below and the classification is associated with the sample input to "label" the sample input.

**Table 1. Beat Classifications**

| Classification | Definition |
|---|---|
| N | Any beat that does not fall into any other classification. |
| B | A bundle branch block beat (usually, it can be the left or right side. |
| S | A supraventricular ectopic beat ("SVEB"); an atrial or nodal (junctional) premature or escape beat; or an aberrated atrial premature beat. |
| V | A ventricular ectopic beat ("VEB"); a ventricular premature beat; an R-on-T ventricular premature beat, or a ventricular escape beat. |
| F | A fusion of a ventricular and a normal beat. |
| Q | A pace beat, a fusion of a paced and a normal beat, or a beat that cannot be classified. |

As mentioned above, in one particular embodiment not shown, the deep neural network comprises eight layers, each layer including 13 operators in 13!+13 summed pairings. In this particular embodiment, the number of labeled samples is as set forth in Table 2 below. Each labeled sample in this particular embodiment has a length of 133 sample points. Of these labeled samples, 75% are randomly chosen and used for training while the remaining 25% are used for validation.

**Table 2. Number of Samples by Classification**

| Classification | Number |
|---|---|
| N | 66,666 |
| V | 61,922 |
| F | 3,970 |
| G | 56,638 |
| Q | 27,582 |
| | |

Referring now to Figure 4A-Figure 4B collectively, the accessed (at 410) sample inputs are then used in performing (at 415) a two-step neural architecture search shown in Figure 4B. As a preliminary matter, however, this particular embodiment first reuses the continuous relaxation function represented by Eqs. (1)-(2). In Eqs. (1)-(2), *k*≡ the number of the weight or operator and *m*≡ the total number of paired operators in a given layer. $O = {\sum }_{k = 1}^{m} w \left(k\right) \left[O1\left(k\right)+O2\left(k\right)\right]$ (1) ${\sum }_{k = 1}^{m} w \left(k\right) = 1$ (2)

Performing (at 415) the two-step neural architecture search using the accessed (at 410) sample inputs includes training (at 417) the plurality of operators within each of the layers and training (at 418) a plurality of weights. Each weight being applied to a respective combination of operators within a respective layer. More particularly, the weights are fixed while the operators are trained (at 417) using the randomly selected 75% of the sample inputs. The operators are then fixed while the weights are trained (at 418) using the same randomly selected 75% of the sample inputs. Note that the order in which the operators are trained (at 417) and the weights are trained (at 418) relative to one another is not material.

Referring once again to Figure 4A, the method 400 then selects (at 420) for each respective layer the operators that yield the maximum weighted output for each respective layer in the two-step neural architecture search. This will typically include deselecting the other paired operators that do not yield the maximum weighted output. Stated more technically, once the training (at 415) is finished, the network architecture can be finalized by retaining the strongest predecessors for each block (with the strength from the weights) and then choose the most likely operators by taking the "argmax"*-i.e.,* determining the block that gives the maximum value for the respective layer. Once the network architecture is finalized, the artificial neural network can then be validated using the 25% of the input samples not used in the training.

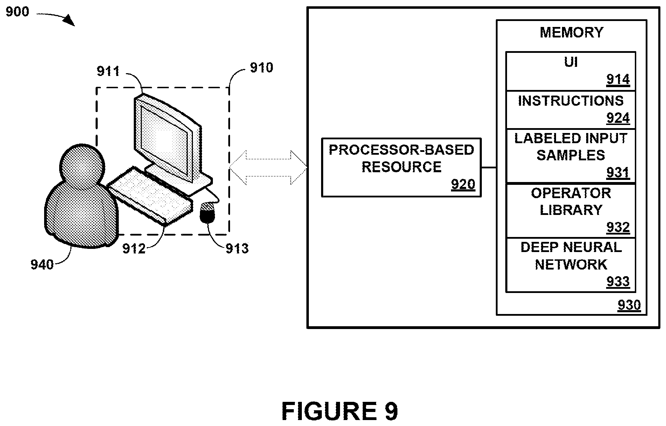

As noted above, a neural architecture search is an automated process. Figure 9 schematically depicts one particular embodiment of a computing apparatus 900 by which the method of Figure 4A-Figure 4B may be performed in the design of the artificial neural network of Figure 5. The computing apparatus 900 may be, for instance, a workstation. The computing apparatus 900 generally comprises, in this particular embodiment, a user interface 910, a processor-based resource 920, and a memory 930. The user interface 920 includes a display 911, one or more peripheral devices such as a keyboard 912 and/or mouse 913, and a software component ("Ul") 914 residing on the memory 930. The Ul software component 914 is executed by the processor-based resource 920 to provide a presentation (not separately shown) on the display 911 with which a user 940 may interact using the peripheral component(s).

The processor-based resource 920 executes machine executable instructions 924 residing in the memory 930 to perform the functionality of the technique described herein. The instructions 924 may be embedded as firmware in the memory 930 or encoded as routines, subroutines, applications, *etc.* The memory 930 is a computer readable non-transitory computer readable medium and may be local or remote. Additionally, the memory 930 has residing therein a library of labeled input samples 931, a library of operators 932, and a deep neural network 933.

Accordingly, in the illustrated embodiment, the computing apparatus 900 performs the software-implemented functionality of the presently disclosed technique. More particularly, the processor-based resource 920 executes the instructions 924, both shown in Figure 9, to perform the programmed functionality of the disclosed neural architecture search technique disclosed herein well as the design of the deep neural network 933. The design of the deep neural network 933 may be performed with input from the user 940 through the user interface 910. The neural architecture search technique may be invoked by the user 940 through the user interface 910 as a part of the design process for the artificial neural network 100.

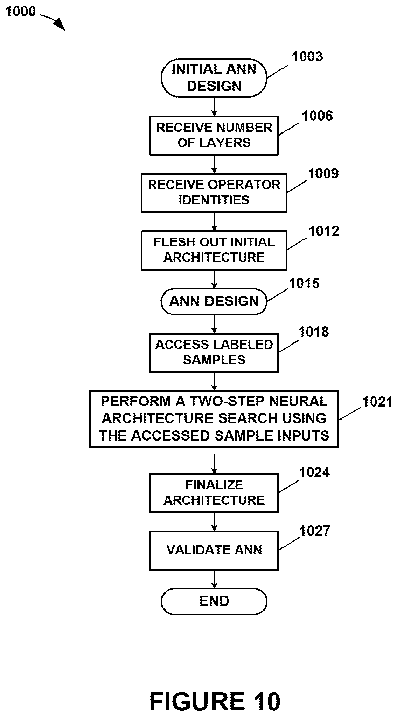

Referring collectively now to Figure 5 and Figure 9, in one particular embodiment, the processor-based resource 920 executes the instructions 924 to perform the workflow 1000 shown in Figure 10. The workflow 1000 begins with the initial design (at 1003) of the initial architecture artificial neural network ("ANN") 500 in Figure 5. As noted above, the artificial neural network 500 is a deep neural network and will be referred to as such moving forward.

This initial design (at 1003) begins with receiving (at 1006) the number of layers and receiving at (1006) the identity of the operators 01-03 (at 1009). This may be accomplished by receiving input from the user 940 through the user interface 910 in Figure 9. The operators 01-03 may be identified from the contents of the operator library 932 presented to the user 940 through the user interface 910 in some manner. Or, they may be identified through manual input or definition by the user 940. The processor-based resource 920 executing the instructions 924 then fleshes out (at 1012) the initial architecture of the deep neural network 500 as shown in Figure 5.

Note that the initial design (at 1003) of the deep neural network 500 may be performed in a more automated fashion in some embodiments. For example, the definition of the initial architecture may be stored in an electronic file (not shown) residing in the memory 930. The processor-based resource 920, under execution of the instructions 924, may access the electronic file to receive the initial definition of the architecture. The processor-based resource 920 can then access the identified operators from the operator library 932 and flesh out the initial architecture.

The processor-based resources 920 then begins (at 1015) the artificial neural network design. This may occur automatically without human intervention upon completing the initial design (at 1003) or invoked by the user 940 through the user interface 910. This artificial neural network design (at 1015) begins by accessing (at 1018) the labeled samples 931. The accessed (at 1018) samples are then used to perform (1021) a two-step neural architecture search as described above. This generally includes first training the plurality of operators within each of the layers while the weights are fixed and then training the weights.

The processor-based resource 920 then finalizes (at 1024) the architecture. This finalization (at 1024) generally includes selecting for each respective layer the operators that yield the maximum weighted output for each respective layer in the two-step neural architecture search while deselecting the other paired operators that do not yield the maximum weighted output. Once the network architecture is finalized (at 1024), the artificial neural network is then validated (at 1027), both also as described above.

An artificial neural network designed using the two-step neural network shows improved performance relative to conventional approaches in terms of outcome accuracy. One particular embodiment was mentioned above in which the artificial neural network computational resource consumption, and design time comprises eight layers, each layer including 13 operators in 13!+13 summed and weighted pairings. In this particular embodiment, the input samples were labeled with the classification set forth in Table 1 and the number of labeled samples in each classification is set forth in Table 2. Each labeled sample in this particular embodiment has a length of 133 sample points. In this embodiment, after 100 epochs, the training accuracy is approximately 97.7% and validation accuracy is approximately 96.8%.

The technique as disclosed herein presumes that a set of input samples has previously been acquired. The input samples in the disclosed embodiments are ECG waveforms. However, it is to be understood that the this is for illustration only, and that embodiments may also operate on input samples of waveforms acquired using other processes. For example, it is common in patient care to monitor a number of physiological characteristics of a patient that can be acquired using other procedures and represented as a waveform. The technique described herein may be used in conjunction with such other waveforms acquired by other procedures and representative of other physiological characteristics. Examples of such other physiological characteristics include, but are not limited to invasive pressures (e.g., invasive blood pressure), gas output from respiration (e.g., oxygen, carbon dioxide), blood oxygenation, internal pressures (e.g., intracranial pressures), measurement of concentration of anesthesia agents (e.g., nitrous oxide), etc.

Furthermore, the input samples disclosed herein are "organic" in the sense that they have been acquired by actually perform ECG procedures on patient(s). The input samples in other embodiments may be synthetic in the sense that they have been acquired by artificially generating them. Still other embodiments may use a combination of organic and synthetic waveforms.

ECG waveforms are acquired over time and represent a number of heartbeats occurring within a predetermined window of time. These waveforms may be sampled as portions of the larger ECG waveform, each portion representing a single heartbeat. The ECG waveforms in the input samples of the illustrated embodiment may therefore be referred to as "beats" because they are portions of a larger waveform representing a single heartbeat. Thus, the input samples for the disclosed technique may be obtained by sampling portions of larger waveforms.

As discussed above, the input ECG samples are beat labeled with the classifications set forth in Table 1. One criterion in the selection of the input sample set is that it contain a statistically significant number of each potential outcome. In the illustrated embodiment, there should therefore be a statistically significant number of N, B, S, V, F and Q beats. As discussed above, the beats are waveforms that are actually sets of ordered data.

Note, however, that other embodiments may be used to analyze and/or classify input samples representing some other physiological characteristic. Examples of such other physiological characteristics include, but are not limited to, to invasive pressures (e.g., invasive blood pressure), gas output from respiration (e.g., oxygen, carbon dioxide), blood oxygenation, internal pressures (e.g., intracranial pressures), measurement of concentration of anesthesia agents (e.g., nitrous oxide), *etc.* The classification of the input samples may therefore vary in some embodiments to accommodate aspects of the physiological characteristic of interest to a clinician. The number of classifications might also vary to as few as two to more than the six seen herein. Still other physiological characteristics and classifications may become apparent to those skilled in the art having the benefit of this disclosure.

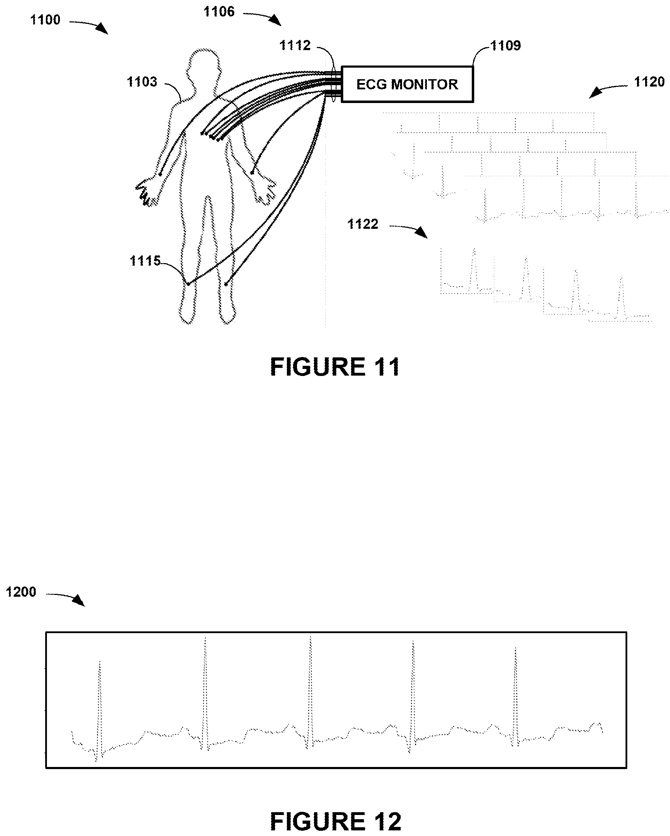

Although the technique as disclosed presumes the input samples will previously have been acquired, acquisition of input samples in an ECG context will now be discussed for the sake of completeness. Figure 11 illustrates an ECG procedure 1100 in accordance with the present disclosure. In Figure 11, a patient 1103 is undergoing the ECG procedure 1100 being administered using the ECG system 1106. The ECG system 1106 comprises an ECG monitor 1109, a plurality of electrical leads 1112, and a plurality of ECG electrodes 1115 (only one indicated). There need not be a 1:1 correspondence between the ECG electrodes 1115 and the electrical leads 1112 as is shown in Figure 11. One common configuration, and one with which the currently disclosed technique may be practiced, is a 10 electrode, 12 lead configuration to measure 10 voltages across a person's body.

The ECG system 1106 acquires a number of ECG waveforms 1120 such as the example waveform 1200 in Figure 1. The ECG waveforms 1120 are then processed to generate the individual input samples, or beats, 1122. The processing may be performed by the ECG monitor 109. However, more likely, the ECG waveforms 1120 will be exported to another computing apparatus or computing system where the processing is performed. The processing may be performed manually on, for example, a workstation. In other embodiments the processing may be performed automatically be a neural network (now shown) trained for beat detection in an ECG waveform. Again, the ECG waveforms 1120 and the input samples 1122 are shown rendered for human perception in Figure 11.

Once acquired, the sample beats are labeled. The practice of the technique disclosed herein is indifferent as to the manner in which the input samples are labeled. In some embodiments, the input sample beats may be manually labeled by, for example, the user 940 using the computing apparatus 900 in Figure 9. The user 940 may be, depending on the task being performed and the skills involved, a person trained and skilled at classifying the input samples. For example, in embodiments such as the one disclosure herein in which the input samples are beats, the user 940 may be clinician or technician who can classify and label input samples as described above.

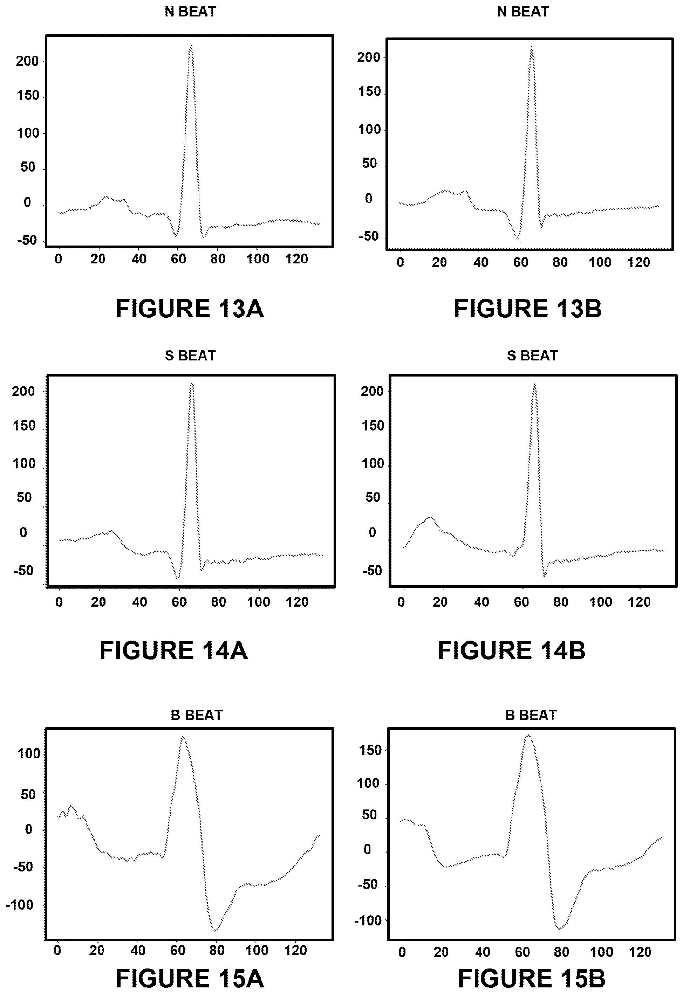

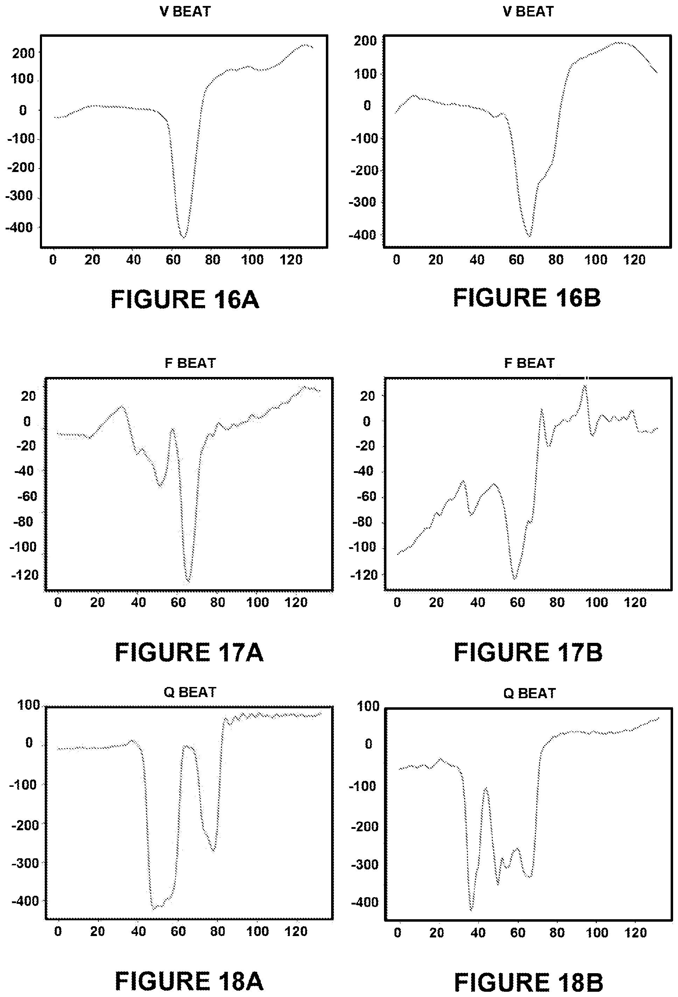

The N, B, S, V, F and Q beat classifications are defined in Table 1. Example beats of each are presented in Figure 13A-Figure 18B. More particularly, Figure 13A-Figure 13B present an example N beat; Figure 14A-Figure 14B present an example B beat; Figure 15A-Figure 15B present an example S beat; Figure 16A-Figure 16B present an example V beat; Figure 17A-Figure 17B present an example F beat; and Figure 18A- Figure 18B present an example Q beat.

Once an artificial neural network has been designed using the two-step neural architecture search disclosed herein it may then be deployed for classification of inputs similar in nature to those on which it is trained. For example, the disclosed embodiments, having been trained on ECG samples classed as N, B, S, V, F and Q beat they may be used to class additional beats as N, B, S, V, F and Q beats in a clinical setting. The improved output accuracy of the disclosed embodiments will directly translate into the clinical setting.

Accordingly, the presently disclosed neural architecture search improves the preexisting technology of artificial neural network design and implementation. The improvement translates into the preexisting technology of whatever pursuit the trained artificial neural network is employed. Furthermore, these are practical applications for the two-step neural architecture search at the heart of the disclosed embodiments.

The discussion above of the computing technology associated with the disclosed technique includes repeated reference to one or more "processor-based resource(s)". Examples include the processor-based resource 320 in Figure 3 and the processor-based resource 920 in Figure 9. As those in the art having the benefit of this disclosure will appreciate, the term "processor" is understood in the art to have a definite connotation of structure. A processor may be hardware, software, or some combination of the two.

In the illustrated embodiments of Figure 3 and Figure 9, the processor-based resources 320, 920 are programmed hardware processors, such as controllers, microcontrollers, or Central Processing Units ("CPUs"). However, in alternative embodiments, the processor-based resources 320, 920 may be Digital Signal Processors ("DSPs"), graphics processors, processor chip sets, Application Specific Integrated Circuits ("ASICs"), appropriately programmed Electrically Programmable Read-Only Memories ("EPROMs"), appropriately programmed Electrically Erasable, Programmable Read-Only Memories ("EEPROMs"), or any other equivalent integrated or discrete logic circuitry, as well as any combinations of such components and one or more machine learning algorithms. Those skilled in the art may appreciate still other implementations for the processor-based resource(s) of the computing technology disclosed herein.

Similarly, the above discussion of computer technology includes references to a "non-transitory, computer readable medium" more colloquially referred to as "memory" in places and by those skilled in the art. Examples include the memory 330 in Figure 3 and the memory 930 in Figure 9. The memories 330, 930 may be distributed, for example, across a computing cloud. The memories 330, 930 may include Read-Only Memory ("ROM"), Random Access Memory ("RAM"), or a combination of the two. The memories 330, 930 will typically be installed memory but may be removable. The memories 330, 930 may be primary storage, secondary, tertiary storage, or some combination thereof implemented using electromagnetic, optical, or solid-state technologies. Accordingly, the memories 330, 930 may be in various embodiments, a part of a mass storage device, a hard disk drive, a solid-state drive, an external drive (whether disk or solid-state), an optical disk, a magnetic disk, a portable external drive, a jump drive, *etc.*

The computing technologies disclosed above also are all shown as single devices located in a single computing apparatus. However, this is only for ease of illustration. Those in the art having the benefit of this disclosure will appreciate that various aspects of the computing technology may be distributed. The memories 330, 530 and processor-based resources 310, 910 may be distributed, for example, across a computing cloud. For another example, in some embodiments the computing apparatus 300 in Figure 3 and/or the computing apparatus 900 in Figure 9 may be some kind of personal computing apparatus other than a workstation. The computing apparatus 300 and the computing apparatus 900 may also access data over a network such as a Local Area Network ("LAN").

In other embodiments, the computing apparatus 300 and/or the computing apparatus 900 may be a computing system. In such a computing system, the processing and memory resources may be distributed across a cloud, for example, accessed from a workstation or other personal computing device over a public or private network such as the Internet. Still other variations in the computing apparatus 300 and/or the computing apparatus 900 may be realized by those skilled in the art having the benefit of this disclosure.

Although the subject matter has been described in language specific to structural features or methodological acts, it is to be understood that the subject matter of the appended claims is not necessarily limited to the specific features or acts described above. Rather, the specific features and acts described above are disclosed as example forms of implementing at least some of the claims.

Various operations of embodiments are provided herein. The order in which some or all of the operations are described should not be construed to imply that these operations are necessarily order dependent. Alternative ordering will be appreciated having the benefit of this description. Further, it will be understood that not all operations are necessarily present in each embodiment provided herein. Also, it will be understood that not all operations are necessary in some embodiments.

It will be appreciated that layers, features, elements, *etc.,* depicted herein are illustrated with particular dimensions relative to one another, such as structural dimensions or orientations, for example, for purposes of simplicity and ease of understanding and that actual dimensions of the same differ substantially from that illustrated herein, in some embodiments. Moreover, "exemplary" is used herein to mean serving as an example, instance, illustration, *etc.,* and not necessarily as advantageous. As used in this application, "or" is intended to mean an inclusive "or" rather than an exclusive "or". In addition, "a" and "an" as used in this application and the appended claims are generally be construed to mean "one or more" unless specified otherwise or clear from context to be directed to a singular form.

Also, at least one of A and B and/or the like generally means A or B or both A and B. Furthermore, to the extent that "includes", "having", "has", "with", or variants thereof are used, such terms are intended to be inclusive in a manner similar to the term "comprising". Also, unless specified otherwise, "first," "second," or the like are not intended to imply a temporal aspect, a spatial aspect, an ordering, *etc.* Rather, such terms are merely used as identifiers, names, etc. for features, elements, items, *etc.* For example, a first element and a second element generally correspond to element A and element B or two different or two identical elements or the same element.

Furthermore, the phrase "capable of" as used herein is a recognition of the fact that some functions described for the various parts of the disclosed apparatus are performed only when the apparatus is powered and/or in operation. Those in the art having the benefit of this disclosure will appreciate that the embodiments illustrated herein include a number of electronic or electro-mechanical parts that, to operate, require electrical power. Even when provided with power, some functions described herein only occur when in operation. Thus, at times, some embodiments of the apparatus of the presently claimed subject matter are "capable of" performing the recited functions even when they are not actually performing them-i.e., when there is no power or when they are powered but not in operation.

## Claims

1. A computer-implemented method for designing an artificial neural network, wherein the artificial neural network is for classifying an input sample representing a physiological characteristic, the method comprising:
creating (405) a deep neural network (100, 500) comprising a plurality of layers, each of the layers comprising a plurality of operators;
accessing (410) a plurality of sample inputs, wherein the plurality of sample inputs are recorded waveforms representing a physiological characteristic of a human body;
performing (415) a two-step neural architecture search using the plurality of sample inputs, the two-step neural architecture search including:
training (417) the plurality of operators within each of the layers; and
training (418) a plurality of weights, each weight being applied to a respective combination of operators within a respective layer; and
selecting (420) for each respective layer the operators that yield the maximum weighted output for each respective layer in the two-step neural architecture search.

2. The method of claim 1, wherein the number of layers is three and the number of operators is three.

3. The method of claim 1, wherein the number of layers is eight and the number of operators is 13.

4. The method of any of claims 1 to 3, wherein creating the deep neural network (100, 500) includes:
receiving a definition of the number of layers, each layer receiving a layer input and generating a layer output, each layer comprising a plurality of operators, each operator including a plurality of operator parameters;
for each of the plurality of operators within each layer:
pairing each operator with an instance of itself and an instance of each other operator within the layer, each instance of each operator receiving a layer input from the previous layer and generating an operator output;
summing the operator outputs of each pair of operators; and
weighting the summed operator outputs, the sum of the weights equaling 1, to generate a plurality of layer outputs.

5. The method of any of claims 1 to 4, wherein the recorded waveforms are electrocardiogram beats.

6. The method of any of claims 1 to 5, wherein the first step of training the plurality of operators within each of the layers includes fixing each of the weights to train the operators.

7. The method of 6, wherein:
each of the operators includes a plurality of operator parameters; and
the second step of training the weights includes fixing operator parameters includes fixing the operator parameters to train the weights.

8. The method of any of claims 1 to 7, wherein:
each of the operators includes a plurality of operator parameters; and
the second step of training the weights includes fixing operator parameters includes fixing the operator parameters to train the weights.

9. The method of any of claims 1 to 8, wherein:
training the deep neural network (100, 500) on the sample inputs includes deselecting for each layer each set of paired operators that does not yield the maximum weighted output; and/or
each layer is a two branch structure mapping from a one input tensor to a one output tensor.

10. A computer-implemented method for creating a deep neural network (100, 500), wherein the deep neural network (100, 500) is for classifying an input sample representing a physiological characteristic, the method comprising:
defining a deep neural network (100, 500), including:
defining a number of layers, each layer receiving a layer input and generating a layer output, each layer comprising a plurality of operators, each operator including a plurality of operator parameters;
for each of the plurality of operators within each layer:
pairing each operator with an instance of itself and an instance of each other operator within the layer, each instance of each operator receiving a layer input from the previous layer and generating an operator output;
summing the operator outputs of each pair of operators; and
weighting the summed operator outputs, the sum of the weights equaling 1, to generate a plurality of layer outputs;
obtaining a plurality of sample inputs, wherein the samples are recorded waveforms representing a physiological characteristic of a human body; and
training the deep neural network (100, 500) on the sample inputs, the training including:
a first pass in which each of the weights is fixed in order to train the operator parameters; and
a second pass in which the operator parameters are fixed in order to train the weights;
selecting for each layer the paired operators that yield the maximum weighted output to provide the layer output; and
deselecting for each layer each set of paired operators that does not yield the maximum weighted output.

11. The method of claim 10, wherein the number of layers is three and the number of operators is three.

12. The method of claim 10, wherein the number of layers is eight and the number of operators is 13.

13. The method of any of claims 10 to 12, wherein the recorded waveforms are electrocardiogram beats.

14. A computing apparatus, comprising:
a processor-based resource; and
a memory electronically communicating with the processor-based resource and encoded with instructions that, when executed by the processor-based resource, perform the method of any of claims 1 to 9, and/or any of claims 10 to 13.

15. A non-transitory, computer readable medium encoded with instructions that, when executed by a processor-based resource, perform the method of any of claims 1 to 9, and/or any of claims 10 to 13.

## Patentansprüche

1. Computerimplementiertes Verfahren zum Entwerfen eines künstlichen neuronalen Netzes, wobei das künstliche neuronale Netz dem Klassifizieren eines Eingangsabtastwerts dient, der eine physiologische Eigenschaft repräsentiert, wobei das Verfahren Folgendes umfasst:
Erzeugen (405) eines tiefen neuronalen Netzes (100, 500), das mehrere Schichten umfasst, wobei jede der Schichten mehrere Operatoren umfasst;
Zugreifen (410) auf mehrere Abtastwerteingaben, wobei die mehreren Abtastwerteingaben aufgezeichnete Signalformen sind, die eine physiologische Eigenschaft eines menschlichen Körpers repräsentieren;
Ausführen (415) einer zweistufigen Suche einer neuronalen Architektur unter Verwendung der mehreren Abtastwerteingaben, wobei die zweistufige Suche einer neuronalen Architektur Folgendes enthält:
Trainieren (417) der mehreren Operatoren innerhalb jeder der Schichten; und
Trainieren (418) mehrerer Gewichte, wobei jedes Gewicht auf eine jeweilige Kombination von Operatoren innerhalb einer jeweiligen Schicht angewendet wird; und
Auswählen (420) für jede jeweilige Schicht der Operatoren, die die maximale gewichtete Ausgabe für jede jeweilige Schicht in der zweistufigen Suche einer neuronalen Architektur ergeben.

2. Verfahren nach Anspruch 1, wobei die Anzahl der Schichten drei ist und die Anzahl der Operatoren drei ist.

3. Verfahren nach Anspruch 1, wobei die Anzahl der Schichten acht ist und die Anzahl der Operatoren 13 ist.

4. Verfahren nach einem der Ansprüche 1 bis 3, wobei das Erzeugen des tiefen neuronalen Netzes (100, 500) Folgendes umfasst:
Empfangen einer Definition der Anzahl von Schichten, wobei jede Schicht eine Schichteingabe empfängt und eine Schichtausgabe erzeugt, wobei jede Schicht mehrere Operatoren enthält,
wobei jeder Operator mehrere Operatorparameter enthält;
für jeden der mehreren Operatoren innerhalb jeder Schicht:
Koppeln jedes Operators mit einer Instanz von sich selbst und einer Instanz jedes anderen Operators innerhalb der Schicht, wobei jede Instanz jedes Operators eine Schichteingabe von der vorhergehenden Schicht empfängt und eine Operatorausgabe erzeugt;
Summieren der Operatorausgaben jedes Paars von Operatoren; und
Gewichten der summierten Operatorausgaben, wobei die Summe der Gewichte gleich 1 ist, um mehrere Schichtausgaben zu erzeugen.

5. Verfahren nach einem der Ansprüche 1 bis 4, wobei die aufgezeichneten Signalformen Elektrokardiogrammschläge sind.

6. Verfahren nach einem der Ansprüche 1 bis 5, wobei der erste Schritt des Trainierens der mehreren Operatoren innerhalb jeder der Schichten das Festlegen jedes der Gewichte enthält, um die Operatoren zu trainieren.

7. Verfahren nach Anspruch 6, wobei:
jeder der Operatoren mehrere Operatorparameter enthält; und
der zweite Schritt des Trainierens der Gewichte das Festlegen der Operatorparameter enthält das Festlegen der Operatorparameter enthält, um die Gewichte zu trainieren.

8. Verfahren nach einem der Ansprüche 1 bis 7, wobei jeder der Operatoren mehrere Operatorparameter enthält; und
der zweite Schritt des Trainierens der Gewichte das Festlegen der Operatorparameter enthält das Festlegen der Operatorparameter enthält, um die Gewichte zu trainieren.

9. Verfahren nach einem der Ansprüche 1 bis 8, wobei
das Trainieren des tiefen neuronalen Netzes (100, 500) an den Abtastwerteingaben das Abwählen jedes Satzes paarweiser Operatoren, der die maximale gewichtete Ausgabe nicht ergibt, für jede Schicht enthält; und/oder jede Schicht eine Zweigstruktur ist, die von einem einen Eingangstensor auf einen einen Ausgangstensor abbildet.

10. Computerimplementiertes Verfahren zum Erzeugen eines tiefen neuronalen Netzes (100, 500), wobei das tiefe neuronale Netz (100, 500) dem Klassifizieren eines Eingabeabtastwerts dient, der eine physiologische Eigenschaft repräsentiert, wobei das Verfahren Folgendes umfasst:
Definieren eines tiefen neuronalen Netzes (100, 500), wobei das Definieren Folgendes umfasst:
Definieren einer Anzahl von Schichten, wobei jede Schicht eine Schichteingabe empfängt und eine Schichtausgabe erzeugt, wobei jede Schicht mehrere Operatoren umfasst, wobei jeder Operator mehrere Operatorparameter enthält;
für jeden der mehreren Operatoren innerhalb jeder Schicht:
Koppeln jedes Operators mit einer Instanz von sich selbst und einer Instanz jedes anderen Operators innerhalb der Schicht, wobei jede Instanz jedes Operators eine Schichteingabe von der vorhergehenden Schicht empfängt und eine Operatorausgabe erzeugt;
Summieren der Operatorausgaben jedes Paars von Operatoren; und
Gewichten der summierten Operatorausgaben, wobei die Summe der Gewichte gleich 1 ist, um mehrere Schichtausgaben zu erzeugen;
Erhalten mehrerer Abtastwerteingaben, wobei die Abtastwerte aufgezeichnete Signalformen sind, die eine physiologische Eigenschaft eines menschlichen Körpers repräsentieren; und
Trainieren des tiefen neuronalen Netzes (100, 500) an den Abtastwerteingaben, wobei das Training Folgendes enthält:
einen ersten Durchgang, in dem jedes der Gewichte festgelegt wird, um die Operatorparameter zu trainieren; und
einen zweiten Durchgang, in dem die Operatorparameter festgelegt werden, um die Gewichte zu trainieren;
Auswählen der paarweisen Operatoren, die die maximale gewichtete Ausgabe ergeben, für jede Schicht, um die Schichtausgabe bereitzustellen; und
Abwählen jedes Satzes paarweiser Operatoren, der die maximale gewichtete Ausgabe nicht ergibt, für jede Schicht.

11. Verfahren nach Anspruch 10, wobei die Anzahl der Schichten drei ist und die Anzahl der Operatoren drei ist.

12. Verfahren nach Anspruch 10, wobei die Anzahl der Schichten acht ist und die Anzahl der Operatoren 13 ist.

13. Verfahren nach einem der Ansprüche 10 bis 12, wobei die aufgezeichneten Signalformen Elektrokardiogrammschläge sind.

14. Rechenvorrichtung, die Folgendes umfasst:
ein prozessorbasiertes Betriebsmittel; und
einen Speicher, der mit dem prozessorbasierten Betriebsmittel elektronisch kommuniziert und mit Anweisungen codiert ist, die, wenn sie durch das prozessorbasierte Betriebsmittel ausgeführt werden, das Verfahren nach einem der Ansprüche 1 bis 9 und/oder einem der Ansprüche 10 bis 13 ausführen.

15. Nicht transitorisches, computerlesbares Medium, das mit Anweisungen codiert ist, die, wenn sie durch ein prozessorbasiertes Betriebsmittel ausgeführt werden, das Verfahren nach einem der Ansprüche 1 bis 9 und/oder einem der Ansprüche 10 bis 13 ausführen.

## Revendications

1. Procédé mis en œuvre par ordinateur de conception d'un réseau de neurones artificiels, dans lequel le réseau de neurones artificiels est destiné à classifier un échantillon d'entrée représentant une caractéristique physiologique, le procédé comprenant :
la création (405) d'un réseau de neurones profond (100, 500) comprenant une pluralité de couches, chacune des couches comprenant une pluralité de d'opérateurs ;
l'accès (410) à une pluralité d'entrées d'échantillons, la pluralité d'entrées d'échantillons étant des formes d'onde enregistrées représentant une caractéristique physiologique d'un corps humain ;
l'exécution (415) d'une recherche d'architecture neuronale en deux étapes au moyen de la pluralité d'entrées d'échantillons, la recherche d'architecture neuronale en deux étapes incluant :
l'entraînement (417) de la pluralité d'opérateurs à l'intérieur de chacune des couches ; et
l'entraînement (418) d'une pluralité de poids, chaque poids étant appliqué à une combinaison respective d'opérateurs à l'intérieur d'une couche respective ; et
la sélection (420), pour chaque couche respective, des opérateurs qui produisent la sortie pondérée maximale pour chaque couche respective dans la recherche d'architecture neuronale en deux étapes.

2. Procédé selon la revendication 1, dans lequel le nombre de couches est de trois et le nombre d'opérateurs est de trois.

3. Procédé selon la revendication 1, dans lequel le nombre de couches est de huit et le nombre d'opérateurs est de 13.

4. Procédé selon l'une quelconque des revendications 1 à 3, dans lequel la création du réseau de neurones profond (100, 500) inclut :
la réception d'une définition du nombre de couches, chaque couche recevant une entrée de couche et générant une sortie de couche, chaque couche comprenant une pluralité d'opérateurs,
chaque opérateur comportant une pluralité de paramètres d'opérateur ;
pour chacun de la pluralité d'opérateurs au sein de chaque couche :
l'appariement de chaque opérateur avec une instance de lui-même et une instance de chaque autre opérateur au sein de la couche, chaque instance de chaque opérateur recevant une entrée de couche de la couche précédente et générant une sortie d'opérateur ;
la sommation des sorties opérateur de chaque paire d'opérateurs ; et
la pondération des sorties d'opérateurs sommées, la somme des poids étant égale à 1, pour générer une pluralité de sorties de couche.

5. Procédé selon l'une quelconque des revendications 1 à 4, dans lequel les formes d'onde enregistrées sont des battements d'électrocardiogramme.

6. Procédé selon l'une quelconque des revendications 1 à 5, dans lequel la première étape d'entraînement de la pluralité d'opérateurs à l'intérieur de chacune des couches comprend la fixation de chacun des poids pour l'entraînement des opérateurs.

7. Procédé selon la revendication 6, dans lequel :
chacun des opérateurs comporte une pluralité de paramètres d'opérateurs ; et
la deuxième étape d'entraînement des poids inclut la fixation des paramètres d'opérateurs inclut la fixation des paramètres d'opérateurs pour l'entraînement des poids.

8. Procédé selon l'une quelconque des revendications 1 à 7, dans lequel :
chacun des opérateurs comporte une pluralité de paramètres d'opérateurs ; et
la deuxième étape d'entraînement des poids inclut la fixation des paramètres d'opérateurs inclut la fixation des paramètres d'opérateurs pour l'entraînement des poids.

9. Procédé selon l'une quelconque des revendications 1 à 8, dans lequel :
l'entraînement du réseau de neurones profond (100, 500) sur les entrées d'échantillon inclut la désélection, pour chaque couche, de chaque ensemble d'opérateurs appariés qui ne produit pas la sortie pondérée maximale ; et/ou
chaque couche est une structure à deux branches établissant une correspondance entre un tenseur d'entrée et un tenseur de sortie.

10. Procédé mis en œuvre par ordinateur pour la création d'un réseau de neurones profond (100, 500), dans lequel le réseau de neurones profond (100, 500) est destiné à classifier un échantillon d'entrée représentant une caractéristique physiologique, le procédé comprenant :
la définition d'un réseau de neurones profond (100, 500), incluant :
la définition d'un nombre de couches, chaque couche recevant une entrée de couche et générant une sortie de couche, chaque couche comprenant une pluralité d'opérateurs, chaque opérateur comportant une pluralité de paramètres d'opérateurs ;
pour chacun de la pluralité d'opérateurs au sein de chaque couche :
l'appariement de chaque opérateur avec une instance de lui-même et une instance de chaque autre opérateur au sein de la couche, chaque instance de chaque opérateur recevant une entrée de couche de la couche précédente et générant une sortie d'opérateur ;
la sommation des sorties opérateur de chaque paire d'opérateurs ; et
la pondération des sorties d'opérateurs sommées, la somme des poids étant égale à 1, pour générer une pluralité de sorties de couche ;
l'obtention d'une pluralité d'entrées d'échantillons, les échantillons étant des formes d'onde enregistrées représentant une caractéristique physiologique d'un corps humain ; et
l'entraînement du réseau de neurones profond (100, 500) sur les entrées d'échantillons, l'entraînement incluant :
une première passe où chacun des poids est fixé pour l'entraînement des paramètres d'opérateurs ; et
une deuxième passe dans laquelle les paramètres d'opérateurs sont fixés pour l'entraînement des poids ;
la sélection, pour chaque couche, des opérateurs appariés qui produisent la sortie pondérée maximale pour fournir la sortie de couche ; et
la désélection, pour chaque couche, de chaque ensemble d'opérateurs appariés qui ne produit pas la sortie pondérée maximale.

11. Procédé selon la revendication 10, dans lequel le nombre de couches est de trois et le nombre d'opérateurs est de trois.

12. Procédé selon la revendication 10, dans lequel le nombre de couches est de huit et le nombre d'opérateurs est de 13.

13. Procédé selon l'une quelconque des revendications 10 à 12, dans lequel les formes d'onde enregistrées sont des battements d'électrocardiogramme.

14. Équipement informatique, comprenant :
une ressource basée sur un processeur ; et
une mémoire communiquant électroniquement avec la ressource basée sur un processeur et codée avec des instructions qui, lorsqu'elles sont exécutées par la ressource basée sur un processeur, mettent en œuvre le procédé selon l'une quelconque des revendications 1 à 9, et/ou l'une quelconque des revendications 10 à 13.

15. Support non transitoire lisible par ordinateur codé avec des instructions qui, lorsqu'elles sont exécutées par une ressource basée sur un processeur, mettent en œuvre le procédé selon l'une quelconque des revendications 1 à 9, et/ou l'une quelconque des revendications 10 à 13.
